(19) Europäisches Patentamt
European Patent Office
Office européen des brevets

(11) Veröffentlichungsnummer: **0 315 826 B1**

(12) **EUROPÄISCHE PATENTSCHRIFT**

(45) Veröffentlichungstag der Patentschrift: **23.10.91**

(21) Anmeldenummer: **88117803.2**

(22) Anmeldetag: **26.10.88**

(51) Int. Cl.$^5$: **C07D 401/06**, C07D 417/14, C07D 413/14, C07D 403/06, C07D 417/06, C07D 401/14, A01N 43/50, A01N 43/40, A01N 43/78, A01N 43/80, A01N 43/56

# (54) Imidazoline.

(30) Priorität: **06.11.87 JP 279042/87**

(43) Veröffentlichungstag der Anmeldung:
**17.05.89 Patentblatt 89/20**

(45) Bekanntmachung des Hinweises auf die Patenterteilung:
**23.10.91 Patentblatt 91/43**

(84) Benannte Vertragsstaaten:
**BE CH DE FR GB IT LI NL**

(56) Entgegenhaltungen:
**EP-A- 0 154 178**
**EP-A- 0 163 855**
**EP-A- 0 212 600**
**EP-A- 0 259 738**
**EP-A- 0 277 317**
**DE-A- 2 205 745**

**CHEM. & PHARMACEUTICAL BULLETIN, Band 27, Nr. 4, 1. April 1979, Seiten 841-847, Tokyo, JP; A. KOSASAYAMA et al.: "Cyclic guanidines. IV. Synthesis of hypoglycemic N-benzhydryl bicyclic guanidines"**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Shiokawa, Kozo**
**210-6, Shukugawara Tama-ku**
**Kawasaki-shi Kanagawa-ken(JP)**
Erfinder: **Tsuboi, Shinichi**
**3-26-1, Hirayama**
**Hino-shi Tokyo(JP)**
Erfinder: **Sasaki, Shoko**
**1-7-3, Higashi-Hirayama**
**Hino-shi Tokyo(JP)**
Erfinder: **Moriya, Koichi**
**5-7-11, Ueno**
**Taito-ku Tokyo(JP)**
Erfinder: **Hattori, Yumi**
**598, Kobiki-cho**
**Hachioji-shi Tokyo(JP)**
Erfinder: **Shibuya, Katsuhiko**
**39-15,Namiki-cho**
**Hachioji-shi Tokyo(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

CHEM. & PHARMACEUTICAL BULLETIN, Band 26, Nr. 12, 1. Dezemeber 1978, Tokyo, JP; F. ISHIKAWA et al.: "Cyclic Guanidines. II. Synthesis of hypoglycemic acyl or alkyl derivatives: of 1-substituted 2-imino-1,3-diazacycloalkane"

CHEM. & PHARMACEUTICAL BULLETIN, Band 26, Nr. 12, 1 Dezember 1978, Seiten 3658-3665, Tokyo, JP; F. ISHIKAWA et al.: "Cyclic guanidines. I. Synthesis of hypoglycemic 1-substituted 2-imino-1,3-diazacycloalkanes"

(74) Vertreter: Schumacher, Günter, Dr. et al
c/o Bayer AG Konzernverwaltung RP Patentabteilung
W-5090 Leverkusen 1 Bayerwerk(DE)

## Beschreibung

Die vorliegende Erfindung betrifft neue Imidazoline, mehrere Verfahren zu ihrer Herstellung und ihre Verwendung als Insektizide.

Es ist bereits bekannt, daß bestimmte 1-substituierte 1,2-Dihydro-2-nitro-iminopyridine antiinflammatorische Wirkungen aufweisen {siehe J. Med. Chem. Band 14, Seiten 988-990 (1971)}.

Nunmehr wurden neue Imidazoline der Formel (I)

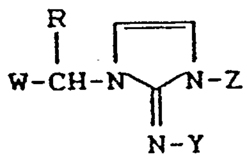

(I)

gefunden, in der

R Wasserstoff oder Methyl ist,

Y Nitro ist,

W eine 5- oder 6-gliedrige heterocyclische Gruppe ist, die 1 oder 2 aus der aus Stickstoff-, Sauerstoff- und Schwefel-Atomen bestehenden Gruppe ausgewählte Hetero-Atome enthält, mit der Maßgabe, daß wenigstens eines der Hetero-Atome ein Stickstoff-Atom ist und die heterocylische Gruppe gegebenenfalls wenigstens einen Substituenten haben kann, der aus der aus Halogen, Alkyl mit 1 bis 4 Kohlenstoff-Atomen, Alkoxy mit 1 bis 4 Kohlenstoff-Atomen, Halogenoalkyl mit 1 bis 4 Kohlenstoff-Atomen, Alkenyl mit 2 bis 4 Kohlenstoff-Atomen Alkylthio mit 1 bis 4 Kohlenstoff-Atomen, Alkylsulfinyl mit 1 bis 4 Kohlenstoff-Atomen, Alkylsulfonyl mit 1 bis 4 Kohlenstoff-Atomen und Alkinyl mit 3 bis 4 Kohlenstoff-Atomen bestehenden Gruppe ausgewählt ist, und

Z Alkyl mit 1 bis 4 Kohlenstoff-Atomen, Alkenyl mit 3 Kohlenstoff-Atomen, Alkinyl mit 3 Kohlenstoff-Atomen, Alkoxyalkyl mit insgesamt 2 bis 4 Kohlenstoff-Atomen, Alkylthioalkyl mit insgesamt 2 bis 4 Kohlenstoff-Atomen, Cyano-substituiertes Alkyl mit 1 bis 2 Kohlenstoff-Atomen, Chloroalkyl mit 3 Kohlenstoff-Atomen, Benzyl (das gegebenenfalls durch Chlor oder Cyano substituiert sein kann), Acetyl, Benzoyl oder $-CH_2-W$ (worin W die im Vorstehenden angegebenen Bedeutungen hat) ist.

Die Imidazoline der Formel (I) werden erhalten, wenn

(a) Verbindungen der Formel (II)

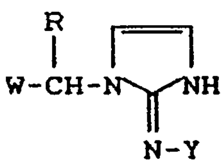

(II)

in der

R, Y, und W die im vorstehenden angegebenen Bedeutungen haben,

mit Verbindungen der Formel (III)

Z-M (III),

in der

Z die im Vorstehenden angegebenen Bedeutungen hat und

M Halogen oder $-OSO_2-L$ bezeichnet, worin L Methyl, Phenyl oder Tolyl darstellt,

in Gegenwart inerter Lösungsmittel und gegebenenfalls in Gegenwart von Basen umgesetzt werden, oder

(b) Verbindungen der Formel (IV)

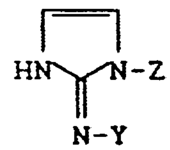

(IV)

in der
Y und Z die im vorstehenden angegebenen Beudeutungen haben,
mit Verbindungen der Formel (V)

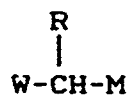

(V),

in der
R, W und M die im vorstehenden angegebenen Bedeutungen haben,
in Gegenwart inerter Lösungsmittel und gegebenenfalls in Gegenwart von Basen umgesetzt werden.

Die neuen Imidazoline zeigen potente insektizide Eigenschaften. Überraschenderweise zeigen die erfindungsgemäßen Imidazoline beträchtlich stärkere insektizide Wirkungen als die bekannten und als antiinflammatorisch wirksam beschriebenen Verbindungen aus dem obengenannten Stand der Technik.

Unter den Imidazolinen der Formel (I) gemäß der vorliegenden Erfindung sind bevorzugte Verbindungen solche, in denen

R Wasserstoff ist,
Y Nitro ist,
W Pyridyl oder Thiazolyl ist, das gegebenenfalls am Ring mit Chlor, Methyl oder Trifluoromethyl substituiert ist, und
Z Methyl, Allyl, Propargyl, 3-Cyanobenzyl, 4-Chlorobenzyl, Acetyl, Benzoyl, 2-Chloropyridin-5-ylmethyl oder 2-Chlorothiazol-5-ylmethyl ist.

Wenn in dem Verfahren (a) beispielsweise 3H-1-(2-Chloropyridin-5-ylmethyl)-2-nitroiminoimidazolin und Methyliodid als Ausgangsstoff eingesetzt werden, kann der Reaktionsverlauf durch die folgende Gleichung dargestellt werden:

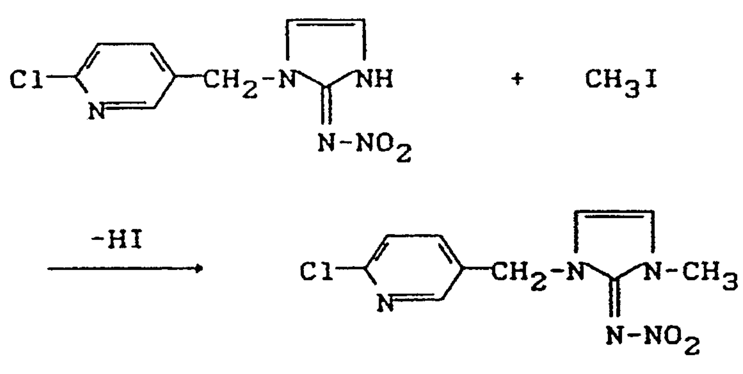


Wenn in dem Verfahren (b) beispielsweise 3H-1-(3-Cyanobenzyl)-2-nitroiminoimidazolin und 2-Chloro-5-(chloromethyl)pyridin als Ausgangsstoffe eingesetzt werden, kann der Reaktionsverlauf durch die folgende Gleichung dargestellt werden:

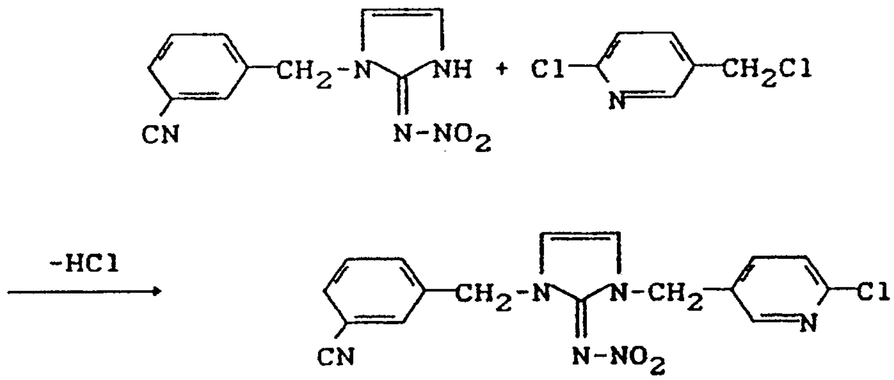


In dem Verfahren (a) sind die Verbindungen der Formel (II) Verbindungen auf der Basis der vorstehenden Definitionen von R, Y, und W, vorzugsweise Verbindungen auf der Basis der vorstehenden bevorzugten Definitionen.

Die Verbindungen der Formel (II) können erhalten werden, wenn 2-Nitroaminoimidazol der Formel

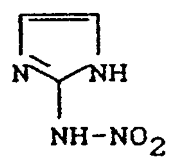

mit den oben bezeichneten Verbindungen der Formel (V) in Gegenwart inerter Lösungsmittel umgesetzt wird.

2-Nitroaminoimidazol kann dadurch erhalten werden, wie in einem nachfolgenden Beispiel gezeigt wird, daß der bekannte S-Methyl-N-nitro-isothioharnstoff mit Aminoacetaldehyd-dimethylacetal in Gegenwart inerter Lösungsmittel umgesetzt wird.

In dem Verfahren (a) sind die Verbindungen der Formel (III) Verbindungen auf der Basis der vorstehenden Definitionen von Z und M.

In der Formel (III) hat Z vorzugsweise die bereits im Vorstehenden als bevorzugt angegebenen Bedeutungen, und M bezeichnet vorzugsweise Chlor, Brom, Iod oder Tosyloxy.

Die Verbindungen der Formel (III) sind auf dem Gebiet der organischen Chemie bereits bekannt, und als Beispiele für diese Verbindungen erwähnt seien
Methyliodid, 3-Cyano-benzylchlorid, 2-Chloro-5-(chloromethyl)pyridin und Acetylchlorid.

In den Verfahren (b) sind die Verbindungen der Formel (IV) Verbindungen auf Basis der vorstehenden Definitionen von Y und Z, vorzugsweise Verbindungen auf der Basis der vorstehenden bevorzugten Definitionen.

Die Verbindungen der Formel (IV) können dadurch erhalten werden, daß 2-Nitroaminoimidazol mit den Verbindungen der Formel (III) in Gegenwart inerter Lösungsmittel umgesetzt wird.

Die Verbindungen der Formel (V) sind Verbindungen auf Basis der vorstehenden Definitionen von R, W und M.

In der Formel (V) haben R und W vorzugsweise die bereits im Vorstehenden als bevorzugt angegebenen Bedeutungen, während M vorzugsweise Chlor, Brom, Iod oder Tosyloxy bezeichnet.

Die Verbindungen der Formel (V) sind bekannt, und als Beispiele für diese Verbindungen erwähnt seien 2-Chloro-5-(chloromethyl)pyridin, 2-Chloro-5-(chloromethyl)thiazol, 5-Chloromethyl-3-methyl-isoxazol und 5-Chloromethyl-2-methylpyridin.

Beispiele für die Lösungsmittel oder Verdünnungsmittel sind Wasser; aliphatische, cycloaliphatische und aromatische, gegebenenfalls chlorierte, Kohlenwasserstoffe, etwa Hexan, Cyclohexan, Petrolether, Ligroin, Benzol, Toluol, Xylol, Methylenchlorid, Chloroform, Kohlenstofftetrachlorid, Ethylenchlorid, Chlorbenzol und dergleichen; Ether, etwa Diethylether, Methylethylether, Diisopropylether, Dibutylether, Propylenoxid, Dioxan, Tetrahydrofuran und dergleichen; Ketone, etwa Aceton, Methylethylketon, Methylisopropylketon, Methylisobutylketon und dergleichen; Nitrile, etwa Acetonitril, Propionitril Acrylnitril und dergleichen; Alkohole, etwa Methanol, Ethanol, Isopropanol, Butanol, Ethylenglycol und dergleichen; Ester, etwa Ethylacetat, Amylacetat und dergleichen; Säureamide, etwa Dimethylformamid, Dimethylacetamid und dergleichen; und Sulfone und Sulfoxide, etwa Dimethylsulfoxid, Sulfolan und dergleichen; sowie Basen, beispielsweise Pyridin etc..

Weiterhin können als Basen anorganische Basen wie Natriumhydroxid, Kaliumcarbonat, Natriumhydrid und dergleichen und organische Basen wie Triethylamin und dergleichen eingesetzt werden.

Bei dem Verfahren (a) kann die Reaktionstemperatur innerhalb eines breiten Bereichs variiert werden. Im allgemeinen wird die Reaktion bei einer Temperatur von etwa 0 °C bis 120 °C, vorzugsweise von etwa 20 °C bis 80 °C, durchgeführt. Im allgemeinen läßt man die Reaktion unter normalem Druck ablaufen, obwohl es auch möglich ist, einen höheren oder niedrigeren Druck anzuwenden.

Wenn das Verfahren (a) gemäß der vorliegenden Erfindung durchgeführt wird, werden beispielsweise etwa 1,0 bis 1,2 mol Triethylamin als Base und etwa 1 bis 1,2 mol, vorzugsweise etwa 1 mol, der Verbindungen der Formel (III) pro 1 mol der Verbindungen der Formel (II) eingesetzt. Die Reaktion kann in Gegenwart eines inerten Lösungsmittels wie Dimethylformamid durchgeführt werden, um die angestrebten Verbindungen der Formel (I) zu erhalten.

Bei der Durchführung des Verfahrens (a) ist es weiterhin möglich, zunächst die Verbindungen der Formel (II) mit einer Base wie Natriumhydrid in die Natrium-Salze der Verbindungen (II) zu überführen, die dann mit den Verbindungen der Formel (III) umgesetzt werden, um praktisch die gewünschten Verbindungen der Formel (I) zu erhalten.

Wenn das oben bezeichnete Verfahren (b) durchgeführt wird, ist es möglich, irgendein inertes Lösungsmittel einzusetzen, das dem im Verfahren (a) gemäß der vorliegenden Erfindung verwendeten ähnelt.

Das Verfahren (b) kann unter den gleichen Reaktionsbedingungen durchgeführt werden, wie sie bei dem Verfahren (a) angewandt wurden. Wenn das Verfahren (b) durchgeführt wird, werden beispielsweise etwa 1,0 bis 1,2 mol Triethylamin als Base und etwa 1 bis 1,2 mol, vorzugsweise etwa 1 mol, der Verbindungen der Formel (V) pro 1 mol der Verbindungen der Formel (IV) eingesetzt. Diese Reaktion sollte vorzugsweise in Gegenwart eines inertes Lösungsmittel wie Ethanol durchgeführt werden, um die angestrebten Verbindungen der Formel (I) zu erhalten.

Die vorliegende Erfindung betrifft weiterhin ein Verfahren zur Bekämpfung schädlicher Insekten, das dadurch gekennzeichnet ist, daß man Imidazoline der Formel (I) auf die schädlichen Insekten und/oder deren Lebensraum einwirken läßt.

Die erfindungsgemäßen Wirkstoffe (Imidazoline der Formel (I)) werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land- und Forstwirtschaft auftreten zum Schutz von Lagerprodukten und Materialien und auf dem Gebiet der Hygiene. Sie sind gegenüber normal empfindlichen und resistenten Species sowie gegen alle oder einige Entwicklungsstadien wirksam. Zu den oben genannten Schädlingen zählen:
Aus der Klasse der Isopoda beispielsweise
oniscus asellus, Armadillidium vulgare und Porcellio scaber;
aus der Klasse der Diplopoda beispielsweise
Blaniulus guttulatus;
aus der Klasse der Chilopoda beispielsweise
Geophilus carpophagus und Scutigera spec.;
aus der Klasse der Symphyla beispielsweise
Scutigerella immaculata;
aus der Ordnung der Thysanura beispielsweise
Lepisma saccharina;
aus der Ordnung der Collembola beispielsweise
Onychiurus armatus;
aus der Ordnung der Orthoptera beispielsweise
Blatta orientalis, Periplaneta americana, Leucophaea maderae, Blatella germanica, Acheta domesticus, Gryllotalpa spp., Locusta migratoria migratorioides, Melanoplus differentialis und Schistocerca gregaria;
aus der Ordnung der Dermaptera beispielsweise
Forficula auricularia,
aus der Ordnung der Isoptera beispielsweise
Reticulitermes spp.;
aus der Ordnung der Anoplura beispielsweise
Phylloxera vastatrix, Pemphigus spp., Pediculus humanus corporis, Haematopinus spp. und Linognathus spp.,
aus der Ordnung der Mallophaga beispielweise

Trichodectes spp. und Damalinea spp.;
aus der Ordnung der Thysanoptera beispielsweise
Hercinothrips femoralis und Thrips tabaci;
aus der Ordnung der Heteroptera beispielsweise
Eurygaster spp., Dysdercus intermedius, Piesma quadrata, Cimex lectularius, Rhodnius prolixus und Triatoma spp.;
aus der Ordnung der Homoptera beispielsweise
Aleurodes brassicae, Bemisia tabaci. Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp.; Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. und Psylla spp.;
Aus der Ordnung der Lepidoptera beispielsweise
Pectinophora gossypiella, Bupalus piniarius, Cheimatobia brumata, Lithocolletis blancardella, Hyponomeuta padella, Plutella maculipennis, Malacosoma neustria, Euproctischrysoorhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, panolis flammea, prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima und Tortrix viridana;
aus der Ordnung der Coleoptera beispielsweise
Anobium punctatum, Rhizopertha dominica, Acanthosacelides obtectus, Hylotrupes bajulus, Agelastica alni, Leptinotarsa decemlineata, Phaedon cochleariae , Diabrotica spp., Psylliodes chrysocephala, Epilachna varivestis, Atomaria spp., Oryzaephilis surinamensis, Antonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptnus spp., Niptus hololeucus, Gibbium psylloides, Tribolium spp., Tenebrio molitor, Agriotes spp., Conoderus spp., Melolontha melolontha, Amphimallon solstitialis und Costelytra zealandica;
aus der Ordnung der Hymenoptera beispielsweise
Diprion spp., Hoplocampa spp., Lasius spp., Monomorium pharaonis und Vespa spp.;
aus der Ordnung der Diptera beispielsweise
Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., bibio hortulanus, Oscinella frit, Phorbia spp., Pegomyia hyoscyami, Ceratitis capitata, Dacus oleae und Tipula paludosa.

Die erfindungsgemäßen Wirkstoffe können in gebräuchliche Formulierungen überführt werden, wie Lösungen, Emulsionen, Suspensionen, Pulver, Schäume, Pasten, Granulate, Aerosole, mit dem Wirkstoff imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der Wirkstoffe mit Streckmitteln, das heißt, mit flüssigen oder verflüssigten gasförmigen oder festen Verdünnungsmitteln oder Trägern, gegegenenfalls unter Verwendung grenzflächenaktiver Mittel, das heißt, von Emulgatoren und/ oder Dispergiermitteln und/oder schaumbildendenen Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chlorethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalen Druck gasförmig wären, beispielsweise Aerosol-Treibmittel wie hologenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatommeenerde und gemahlene synethetische Minerale wie hochdisperse Kieselsäure, Aluminiumoxid und Silicate. Als feste Träger für Granulate können zerkleinerte und fraktionierte Natursteinmaterialien verwendet werden, etwa Calcit, Marmor, Bimsstein, Sepiolith und Dolomit sowie synthetisches Granulat aus anorganischen und organiscchen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarypolyglycol-Ether, Alkylsulfonate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% des erfindungsgemäßen Wirkstoffs.

Die erfindungsgemäßen Wirkstoffe können in ihren handelsüblichen Formulierungen oder den aus diesen Formulierungen hergestellten Anwendungsform im Gemisch mit anderen aktiven Verbindungen vorliegen, etwa mit Insektiziden, Ködern, Sterilisationsmitteln, Akariziden, Nematoziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die erfindungsgemäßen Wirkstoffe können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst wirksam zu sein.

Der Gehalt des Wirkstoffes in den aus den im Handel erhältlichen Formulierungen hergestellten Anwendungsformen kann innerhalb weiter Grenzen variieren.

Die Konzentration des Wirkstoffs in den Anwendungsformen kann 0.0000001 bis 100 Gew.-% betragen und liegt vorzugsweise zwischen 0,0001 und 1 Gew.-%.

Die Verbindungen werden in üblicher Weise in einer den Anwendungesformen angemessenen Form zur Anwendung gebracht.

Bei der Verwendung gegen Hygiene-Schädlinge und Schädlinge in Produkt-Vorräten zeichnen sich die Wirkstoffe durch eine hervorragende Rückstandswirkung auf Holz und Ton sowie eine gute Beständigkeit gegen Alkali auf gekalkten Unterlagen aus.

Die Herstellung und die Anwendung der erfindungsgemäßen Wirkstoffe sind den folgenden Beispielen zu entnehmen.

## Herstellungsbeispiele

### Beispiel 1

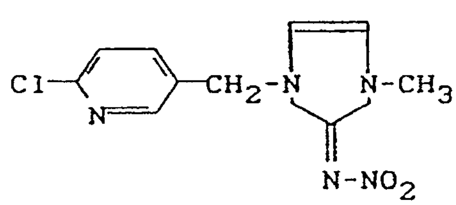

3H-1-(2-Chloropyridin-5-ylmethyl)-2-nitroiminoimidazolin (5,1 g) wurde in trockenem Dimethylformamid (40 ml) gelöst. zu der entstandenen Lösung wurde bei einer Temperatur bis 10°C anteilweise 60-proz. Natriumhydrid in Mineralöl (0,8 g) hinzugefügt. Nach beendeter Zugabe wurde die Reaktionsmischung bei Raumtemperatur gerührt, bis die Entwicklung von Wasserstoff beendet war. Danach wurde Methyliodid (3,4 g) bei der gleichen Temperatur zu der Mischung hinzugefügt, und die Reaktionsmischung wurde 3 Stunden

bei Raumtemperatur gerührt. Nach dieser Reaktionszeit wurde die Reaktionsmischung in Eiswasser gegossen. Mit Hilfe von Dichlormethan wurde eine Extraktion vorgenommen. Die Dichloromethan-Schicht wurde in üblicher Weise behandelt und der erhaltene Rückstand wurde durch Chromatographie an einer Silicagel-Säule gereinigt, wonach die gewünschte Verbindung, d.h. 1-(2-Chloro-pyridin-5-ylmethyl)-3-methyl-2--nitroiminoimidazolin, (3,0 g) erhalten wurde; Schmp. 165-167 °C.

Beispiel 2

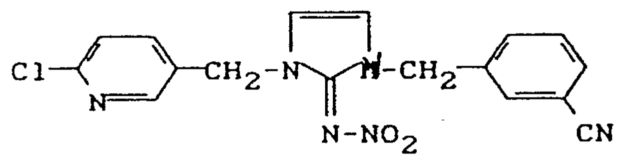

Eine Mischung aus 3H-1-(3-Cyanobenzyl)-2-nitroiminoimidazolin (4,9 g), 2-Chloro-5-chloromethylpyridin (3,2 g), Triethylamin (2,2 g) und Ethanol (40 ml) wurde 3 h unter Rühren zum Rückfluß erhitzt. Nachdem das Ethanol unter vermindertem Druck abdestilliert worden war, wurde der Rückstand mit Dichlormethan vermischt, und die resultierende Mischung wurde mit Wasser und dann mit einer 1-proz. Salzsäure-Lösung gewaschen. Nachdem die Dichloromethan-Schicht in üblicher Weise behandelt worden war, wurde der erhaltene Rückstand durch Chromatographie an einer Silicagel-Säule gereinigt, wonach die gewünschte Verbindung, d.h. 1-(2-Chloro-pyridin-5-ylmethyl)-3-(3-cyanobenzylmethyl)-2-nitro-iminoimidazolin, (0,7 g) erhalten wurde; Schmp. 204-206 °C.

Verbindungen der Formel (I) gemäß der vorliegenden Erfindung, die nach den gleichen Verfahren wie in den Beispielen 1 und 2 hergestellt werden können, sind zusammen mit den Verbindungen der Beispiele 1 und 2 in der folgenden Tabelle 1 aufgeführt.

**Tabelle 1**

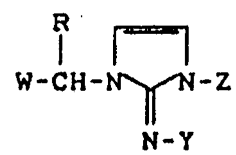

| Verbindung Nr. | R | Y | W | Z | Schmp. |
|---|---|---|---|---|---|
| 1 | H | Nitro | 3-Pyridyl | Methyl | |
| 2 | H | Nitro | 3-Pyridyl | n-Butyl | |
| 3 | H | Nitro | 3-Pyridyl | 2-Methylthioethyl | |
| 4 | H | Nitro | 3-Pyridyl | 2-Cyanoethyl | |
| 5 | H | Nitro | 2-Fluoropyridin-5-yl | Allyl | |
| 6 | H | Nitro | 2-Chloropyridin-5-yl | Methyl | 165-167° C |
| 7 | H | Nitro | 2-Chloropyridin-5-yl | Allyl | |
| 8 | H | Nitro | 2-Chloropyridin-5-yl | Benzoyl | |
| 9 | H | Nitro | 2-Chloropyridin-5-yl | 4-Chlorobenzyl | 196-198° C |

Tabelle 1 (Fortsetzung)

| Verbindung Nr. | R | Y | W | Z | Schmp. |
|---|---|---|---|---|---|
| 10 | H | Nitro | 2-Chloropyridin-5-yl | 3-Cyanobenzyl | 204-206° C |
| 11 | H | Nitro | 2,3-Dichlorpyridin-5-yl | Methyl | |
| 12 | H | Nitro | 2-Methylpyridin-5-yl | 2-Ethoxyethyl | |
| 13 | H | Nitro | 3-Methylisoxazol-5-yl | Methyl | |
| 14 | H | Nitro | 1-Methylpyrazol-4-yl | 3,4-Dichlorobenzyl | |
| 15 | H | Nitro | 1-Isopropylpyrazol-4-yl | Methyl | |
| 16 | H | Nitro | 2-Chlorothiazol-5-yl | Methyl | |
| 17 | H | Nitro | 1,2,5-Thiazdiazol-3-yl | Methyl | |
| 18 | H | Nitro | 2-Methylpyrazin-5-yl | 4-Chlorobenzyl | |
| 19 | H | Nitro | 2-Chloropyridin-5-yl | 2-Chloropyridin-5-ylmethyl | 164-166° C |

Tabelle 1 (Fortsetzung)

| Verbindung Nr. | R | Y | W | Z | Schmp. |
|---|---|---|---|---|---|
| 20 | H | Nitro | 2-Chlorothiazol-5-yl | 2-Chloropyridin-5-ylmethyl | |
| 21 | H | Nitro | 2-Chlorothiazol-5-yl | 2-Chlorothiazol-5-ylmethyl | |
| 22 | H | Nitro | 3-Methylisoxazol-5-yl | 3-Chloropyridin-6-ylmethyl | |

Beispiel 3

Herstellung eines Zwischenprodukts

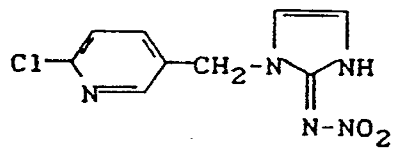

2-Nitroaminoimidazol (12,8 g) wurde in trockenem Dimethylformamid (100 ml) gelöst. Zu der entstandenen lösung wurde bei 5° C anteilweise 60-proz. Natriumhydrid in Mineralöl (4,8 g) hinzugefügt. Nach beendeter Zugabe wurde die Reaktionsmischung bei Raumtemperatur gerührt bis die Entwicklung von Wasserstoff beendet war. Danach wurde eine Lösung von 2-Chloro-5-(chloromethyl)pyridin (16,1 g) in Dimethylformamid (20 ml) bei einer Temperatur bis zu 10° C tropfenweise zu der Reaktionsmischung hinzugefügt. Dann wurde die Reaktionsmischung 3 Stunden bei Raumtemperatur gerührt. Danach wurde die Reaktionsmischung in Eiswasser gegessen, um das gewünschte kristalline Produkt auszufällen. Das Produkt wurde durch Filtration abgetrennt, in Ethanol gewaschen und getrocknet, wonach die gewünschte Verbindung, d.h. 3H-1-(2-Chloropyridin-5-ylmethyl )-2-nitroiminoimidazolin (15,4 g ) erhalten wurde; Schmp. 186-189° C.

Nach der gleichen Arbeitsweise, wie sie im Vorstehenden dargestellt ist, wurde unter Einsatz von 3-Cyanobenzylchlorid an Stelle des 2-Chloro-5-(chloromethyl)pyridins 4H-1-(3-Cyanobenzyl)-2-nitroiminoimidazolin erhalten; Schmp. 193-194° C. (Zersetzung).

Beispiel 4

Herstellung eines Zwischenproduktes

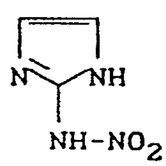

S-Methyl-N-nitro-isothioharnstoff (13,5 g) und Aminoacetaldehyd-dimethylacetal (10,5 g) wurden zusammen in Methanol (150 g) 3 h bei 45° C gerührt. Nach dieser Reaktion wurde 2N HCl (40 ml) zu der Reaktionsmischung hinzugefügt, und die Mischung wurde 4 Stunden bei 60° C gerührt und dann abgekühlt.

Das auf diese Weise gebildete kristalline Produkt wurde durch Filtration abgetrennt, in Methanol gewaschen und getrocknet, wonach die gewünschte Verbindung, d.h. 2-Nitroaminoimidazol (9 g) erhalten wurde; Schmp. 195-200° C (Zersetzung).

Biologische Test-Beispiele

Vergleichs-Verbindung:

C-1:

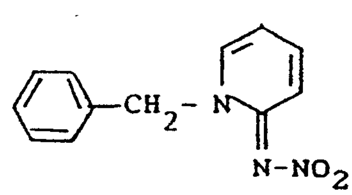

(offenbart in J. Med.. Chem. Band 14, Seiten 988-990 (1971)}

Beispiel 5

Biologischer Test durchgeführt gegen Nephotettix cincticeps, der Resistenz gegenüber Insektiziden der Organo-phosphor-Reihe zeigte

Herstellung einer Test-Formulierung:

| | | |
|---|---|---|
| Lösungsmittel: | 3 Gew.-Teile | Xylol |
| Emulgator: | 1 Gew.-Teil | Polyoxyethylen-alkyl-phenylether |

Zur Herstellung einer geeigneten Formulierung einer aktiven Verbindung wurde 1 Gew.-teil des Wirkstoffs mit der obenbezeichnten Menge Lösungsmittel, das die obenangegebene Menge Emulgator enthielt, vermischt, und die Mischung wurde mit Wasser auf die vorher festgesetzte Konzentration verdünnt.

Test-Verfahren:

Benutzt wurde eine Anzahl Töpfe mit jeweils einem Durchmesser von 12 cm, in die Reispflanzen-Setzlinge mit einer Höhe von etwa 10 cm gesetzt waren.

Auf jedem eingetopften Reispflanzen-Setzling wurden 10 ml einer wäßrigen Lösung des Wirkstoffs mit der vorher festgelegten Konzentration gesprüht.

Nachdem die gesprühte Lösung eingetrocknet war, wurde jeder der Töpfe mit einem Drahtkorb von 7 cm Durchmesser und 14 cm Höhe bedeckt, in dem 30 ausgewachsene weibliche Exemplare von Nephotettix cincticeps, die gegen Insektizide der Organophosphor-Reihe Resistenz zeigten, ausgesetzt wurden, und jeder Topf wurde dann in einen Raum mit konstanter Temmperatur gestellt. Zwei Tage danach wurde die Zahl der toten Insekten ermittelt, um die Mortalitäts-Rate der Insekten zu erhalten.

In diesem Text bewirkten die Verbindungen Nr. 6, 10 und 19 eine Insekten-Mortalität von 100 % bei einer Wirkstoff-Konzentration von 40 ppm, während die Kontrolle C-1 bei diese Konzentration unwirksam war.

Beispiel 6

Biologischer Test durchgeführt gegen Laternenträger

Test-Verfahren:

Benutzt wurde eine Anzahl Töpfe mit jeweils einem Durchmesser von 12 cm, in die Reispflanzen-Setzlinge mit einer Höhe von etwa 10 cm gesetzt waren.

Auf jeden eingetopften Reispflanzen-Setzling wurden 10 ml einer wäßrigen Lösung des Wirkstoffs in ähnlicher Weise wie in Beispiel 5 mit der vorher festgelegten Konzentration gesprüht.

Nachdem die gesprühte Lösung eingetrocknet war, wurde jeder der Töpfe mit einem Drahtkorb vom 7 cm Durchmesser und 14 cm Höhe bedeckt, in dem 30 ausgewachsene weibliche Exemplare von Nilaparvata lugens, die gegen Insektizide der Organophosphor-Reihe Resistenz zeigten, ausgesetzt wurden, und jeder Topf wurde dann in einen Raum mit konstanter Temperatur gestellt. Zwei Tage danach wurde die Zahl der toten Insekten ermittelt, um die Mortalitäts-Rate der Insekten zu erhalten.

In ähnlicher Weise wurden Tests mit Sogatella furcifera und Laodelphax striatella, die gegen Insektizide der Organophosphor-Reihe Resistenz zeigten, zur Ermittlung der Mortalitäts-Rate der Insekten durchgeführt.

In diesem Text bewirkten die Verbindungen Nr. 10 und 19 eine Insekten-Mortalität bei Nilparvata lugens, sogatella furcifera und Laodelphax striatella von 100 % bei einer Wirkstoff-Konzentration von 200 ppm, während die Kontrolle C-1 bei dieser Konzentration unwirksam war.

Beispiel 7

Biologischer Test durchgeführt gegen Myzus persicae, der gegenüber Insektiziden der Organophosphor-

und Carbamat-Reihe Resistenz zeigte

Test-Verfahren:

Auf Auberginen-Setzlinge (schwarze längliche Auberginen) mit jeweils einer Höhe von 20 cm, die in unglasierten Töpfen von 15 cm Durchmesser gezogen worden waren, wurden pro Setzling 200 herangezogene Exemplare von Myzus persicae, der gegenüber Insektiziden der Organophosphor-und Carbamat-reihe Resistenz besaß, ausgesetzt. Einen Tag nach der Inokulierung wurde eine wäßrige Lösung mit einer vorher festgelegten Konzentration des Wirkstoffs, die nach einer Arbeitsweise ähnlich derjenigen von Beispiel 5 hergestellt worden war, in einer genügenden Dosierung mit Hilfe einer Spritzpistole auf die Setzling

Der oben angegebene Test wurde für jede der nachstehend bezeichneten Wirkstoffe mit den angegebenen Konzentrations-Dosierungen durchgeführt. Nach dem Sprühen der insektiziden Lösung wurden die Setzlinge für den Test in jedem Topf 24 h in einem auf einer Temperatur von 28°C gehaltenen Gewächshaus stehen gelassen, und danach wurde die Mortalitäts-Rate der Insekten für jeden Test bestimmt. Der gleiche Test wurde zweimal wiederholt, um eine genaue Bestimmung der Mortalitäts-Rate zu erhalten.

In diesem Test bewirkten die Verbindungen Nr. 6 und 19 eine Insekten-Mortalität von 100 % bei einer Wirkstoff-Konzentration von 200 ppm, während die Kontrolle C-1 sogar bei einer Konzentration von 1000 ppm unwirksam war.

## Patentansprüche

**1.** Imidazoline der Formel (I)

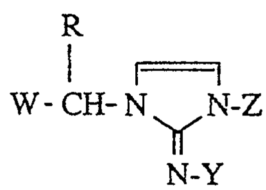

(I)

in der

- R Wasserstoff oder Methyl ist,
- Y Nitro ist,
- W eine 5- oder 6-gliedrige heterocyclische Gruppe ist, die 1 oder 2 aus der aus Stickstoff-, Sauerstoff- und Schwefel-Atomen bestehenden Gruppe ausgewählte Hetero-Atome enthält, mit der Maßgabe, daß wenigstens eines der Hetero-Atome ein Stickstoff-Atom ist, und mit der anderen Maßgabe, daß die heterocylische Gruppe gegebenenfalls wenigstens einen Substituenten haben kann, der aus der aus Halogen, Alkyl mit 1 bis 4 Kohlenstoff-Atomen, Alkoxy mit 1 bis 4 Kohlenstoff-Atomen, Halogenoalkyl, mit 1 bis 4 Kohlenstoff-Atomen, Alkenyl mit 2 bis 4 Kohlenstoff-Atomen, Alkylthio mit 1 bis 4 Kohlenstoff-Atomen, Alkylsulfinyl mit 1 bis 4 Kohlenstoff-Atomen, Alkylsulfonyl mit 1 bis 4 Kohlenstoff-Atomen und Alkinyl mit 3 bis 4 Kohlenstoff-Atomen bestehenden Gruppe ausgewählt ist, und
- Z Alkyl mit 1 bis 4 Kohlenstoff-Atomen, Alkenyl mit 3 Kohlenstoff-Atomen, Alkinyl mit 3 Kohlenstoff-Atomen, Alkoxyalkyl mit insgesamt 2 bis 4 Kohlenstoff-Atomen, Alkylthioalkyl mit insgesamt 2 bis 4 Kohlenstoff-Atomen, Cyanosubstituiertes Alkyl mit 1 bis 2 Kohlenstoff-Atomen, Chloroalkyl mit 3 Kohlenstoff-Atomen, Benzyl (das gegebenenfalls durch Chlor oder Cyano substituiert sein kann), Acetyl, Benzoyl oder $-CH_2-W$ (worin W die im Vorstehenden angegebenen Bedeutungen hat) ist.

**2.** Verbindungen nach Anspruch 1, dadurch gekennzeichnet, daß

- R Wasserstoff ist,
- Y Nitro ist,
- W Pyridyl oder Thiazolyl ist, das gegebenenfalls am Ring mit Chlor, Methyl oder Trifluoromethyl

substituiert ist, und

Z Methyl, Allyl, Propargyl, 3-Cyanobenzyl 4-Chloro-benzyl, Acetyl, Benzoyl, 2-Chloropyridin-5-ylmethyl oder 2-Chlorothiazol-5-ylmethyl ist.

**3.** Verfahren zur Herstellung von Imidazolinen der Formel (I)

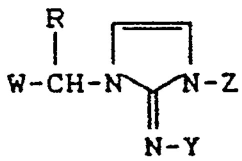

(I)

in der R, Y, W und Z die im Anspruch 1 angegebene Bedeutung besitzen,

dadurch gekennzeichnet, daß

(a) Verbindungen der Formel (II)

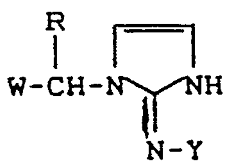

(II)

in der
R, Y und W die im Vorstehenden angegebenen Bedeutungen haben,
mit Verbindungen der Formel (III)

Z-M (III),

in der
Z die im Vorstehenden angegebenen Bedeutungen hat und
M Halogen oder $-OSO_2-L$ bezeichnet, worin L Methyl, Phenyl oder Tolyl darstellt,
in Gegenwart inerter Lösungsmittel und gegegebenefalls in Gegenwart von Basen umgesetzt werden,

oder

(b) Verbindungen der Formel (IV)

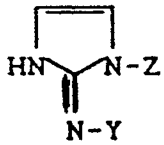

(IV)

in der
Y und Z die im Vorstehenden angegebenen Bedeutungen haben,
mit Verbindungen der Formel (V)

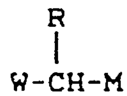 (V),

in der
R, W und M die im Vorstehenden angegebenen Bedeutungen haben,

In Gegenwart inerter Lösungsmittel und gegebenenfalls in Gegenwart von Basen umgesetzt werden.

**4.** Insektizide Formulierungen, dadurch gekennzeichnet, daß sie wenigstens ein Imidazolin der Formel (I) enthalten.

**5.** Verfahren zur Bekämpfung schädlicher Insekten, dadurch gekennzeichnet, daß man Imidazoline der Formel (I) auf die schädlichen Insekten und/oder deren Lebensraum einwirken läßt.

**6.** Verwendung von Imidazolinen der Formel (I) zur Bekämpfung schädlicher Insekten.

**7.** Verfahren zur Herstellung insektizider Formulierungen, dadurch gekennzeichnet, daß Imidazoline der Formel (I) mit Streckmitteln und/oder grenzflächenaktiven Mitteln vermischt werden.

**Claims**

**1.** Imidazolines of the formula (I)

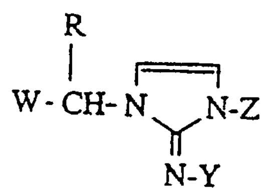 (I)

in which
R is hydrogen or methyl,
Y is nitro,
W is a 5- or 6-membered heterocyclic group which contains 1 or 2 hetero atoms selected from the group comprising nitrogen, oxygen and sulphur atoms, with the proviso that at least one of the hetero atoms is a nitrogen atom, and with the further proviso that the heterocyclic group can optionally have at least one substituent which is selected from the group comprising halogen, alkyl having 1 to 4 carbon atoms, alkoxy having 1 to 4 carbon atoms, halogeno-alkyl having 1 to 4 carbon atoms, alkenyl having 2 to 4 carbon atoms, alkylthio having 1 to 4 carbon atoms, alkylsulphinyl having 1 to 4 carbon atoms, alkylsulphonyl having 1 to 4 carbon atoms and alkinyl having 3 to 4 carbon atoms, and
Z is alkyl having 1 to 4 carbon atoms, alkenyl having 3 carbon atoms, alkinyl having 3 carbon atoms, alkoxyalkyl having a total of 2 to 4 carbon atoms, alkylthioalkyl having a total of 2 to 4 carbon atoms, cyano-substituted alkyl having 1 to 2 carbon atoms, chloroalkyl having 3 carbon atoms, benzyl (which can optionally be substituted by chlorine or cyano), acetyl, benzoyl or $-CH_2$-W (where W has the meanings given above).

**2.** Compounds according to Claim 1, characterised in that
R is hydrogen,
Y is nitro,
W is pyridyl or thiazolyl, each of which is optionally substituted on the ring with chlorine, methyl or trifluoromethyl, and
Z is methyl, allyl, propargyl, 3-cyanobenzyl, 4-chloro-benzyl, acetyl, benzoyl, 2-chloropyridin-5-ylmethyl or 2-chlorothiazol-5-ylmethyl.

**3.** Process for the preparation of imidazolines of the formula (I)

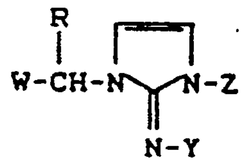

(I)

in which R, Y, W and Z have the meaning given in Claim 1, characterised in that

(a) compounds of the formula (II)

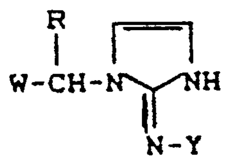

(II)

in which

R, Y and W have the meanings given above,

are reacted with compounds of the formula (III)

Z-M (III)

in which

Z has the meanings given above and

M denotes halogen or $-OSO_2-L$, where L represents methyl, phenyl or tolyl,

in the presence of inert solvents and if appropriate in the presence of bases,

or

(b) compounds of the formula (IV)

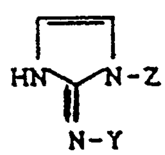

(IV)

in which

Y and Z have the meanings given above,

are reacted with compounds of the formula (V)

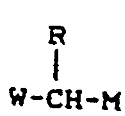

(V)

in which

R, W and M have the meanings given above,

in the presence of inert solvents and if appropriate in the presence of bases.

**4.** Insecticidal formulations, characterised in that they contain at least one imidazoline of the formula (I).

**5.** Method of combating harmful insects, characterised in that imidazolines of the formula (I) are allowed to act on the harmful insects and/or their habitat.

6. Use of imidazolines of the formula (I) for combating harmful insects.

7. Process for the preparation of insecticidal formulations, characterised in that imidazolines of the formula (I) are mixed with extenders and/or surface-active substances.

## Revendications

1. Imidazolines de formule (I)

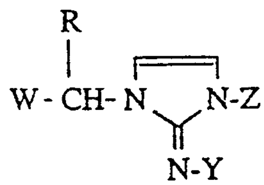

(I)

dans laquelle

R représente un atome d'hydrogène ou un groupe méthyle,
Y représente un groupe nitro,
W représente un groupe hétérocyclique penta- ou hexagonal, qui contient 1 ou 2 hétéro-atomes choisis parmi le groupe comprenant un atome d'azote, un atome d'oxygène et un atome de soufre, à condition qu'au moins un des hétéro-atomes représente un atome d'azote, et à cette autre condition que le groupe hétérocyclique puisse contenir éventuellement au moins un substituant choisi parmi le groupe comprenant un atome d'halogène, un groupe alkyle contenant de 1 à 4 atomes de carbone, un groupe alcoxy contenant de 1 à 4 atomes de carbone, un groupe halogénalkyle contenant de 1 à 4 atomes de carbone, un groupe alcényle contenant de 2 à 4 atomes de carbone, un groupe alkylthio contenant de 1 à 4 atomes de carbone, un groupe alkylsulfinyle contenant de 1 à 4 atomes de carbone, un groupe alkylsulfonyle contenant de 1 à 4 atomes de carbone et un groupe alcynyle contenant de 3 à 4 atomes de carbone, et
Z représente un groupe alkyle contenant de 1 à 4 atomes de carbone, un groupe alcényle contenant 3 atomes de carbone, un groupe alcynyle contenant 3 atomes de carbone, un groupe alcoxyalkyle contenant au total 2 à 4 atomes de carbone, un groupe alkylthioalkyle contenant au total 2 à 4 atomes de carbone, un groupe alkyle substitué par un groupe cyano et contenant de 1 à 2 atomes de carbone, un groupe chloroalkyle contenant 3 atomes de carbone, un groupe benzyle (qui peut éventuellement être substitué par un atome de chlore ou par un groupe cyano), un groupe acétyle, un groupe benzoyle ou $-CH_2-W$ (où W a les significations indiquées ci-dessus).

2. Composés selon la revendication 1, **caractérisés en ce que**

R représente un atome d'hydrogène,
Y représente un groupe nitro,
W représente un groupe pyridyle ou un groupe thiazolyle éventuellement substitué sur le noyau par un atome de chlore, par un groupe méthyle ou par un groupe trifluorométhyle, et
Z représente un groupe méthyle, un groupe allyle, un groupe propargyle, un groupe 3-cyanobenzyle, un groupe 4-chlorobenzyle, un groupe acétyle, un groupe benzoyle, un groupe 2-chloropyridin-5-ylméthyle ou un groupe 2-chlorothiazol-5-ylméthyle.

3. Procédés pour la préparation d'imidazolines de formule (I)

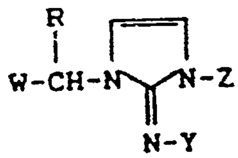

(I)

dans laquelle R, Y, W et Z ont la signification indiquée dans la revendication 1,

**caractérisés en ce que**

(a) on fait réagir des composés de formule (II)

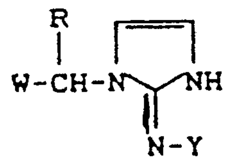

(II)

dans laquelle R, Y et W ont les significations indiquées ci-dessus, avec des composés de formule (III)

Z-M (III)

où
- Z a les significations indiquées ci-dessus et
- M représente un atome d'halogène ou $-OSO_2-L$ où L représente un groupe méthyle, un groupe phényle ou un groupe tolyle,

en présence de solvants inertes et éventuellement en présence de bases,

ou

(b) on fait réagir des composés de formule (IV)

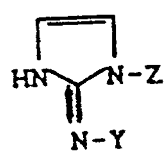

(IV)

dans laquelle Y et Z ont les significations mentionnées ci-dessus, avec des composés de formule (V)

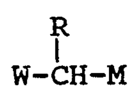

(V)

où
R, W et M ont les significations indiquées ci-dessus

en présence de solvants inertes et eventuellement en présence de bases.

4. Formulations d'insecticides, **caractérisées en ce qu'**elles contiennent au moins une imidazoline de formule (I).

5. Procédé destiné à lutter contre des insectes nuisibles, **caractérisé en ce qu'**on laisse agir des imidazolines de formule (I) sur les insectes nuisibles et/ou dans leur biotope.

6. Utilisation d'imidazolines de formule (I) pour lutter contre des insectes nuisibles.

7. Procédé pour la préparation de formulation insecticides, **caractérisé an ce qu'**on mélange des imidazolines de formule (I) avec des diluants et/ou des agents tensio-actifs.